# EUROPEAN PATENT APPLICATION

(11) **EP 1 495 731 A1**
(43) Date of publication of application: **12.01.2005**
(21) Application number: 02787332.2
(22) Date of filing: 30.12.2002
(51) Int. Cl.: A61B 17/72

(54) **AN INTRAMEDULLARY PIN**

(30) Priority: 29.12.2001 CN 01277174
(71) Applicant: Qian, Benwen, Quieng-Pu, Shanghai 201700 (CN)
(72) Inventor: Qian, Benwen, Quieng-Pu, Shanghai 201700 (CN)
(74) Representative: Laako, Tero Jussi
(86) International application number: PCT/CN2002/000924
(87) International publication number: WO 2003/055399

(57) **Abstract**

The present invention relates to an intramedullary pin which includes a body. Prongs which respectively extend towards different directions are provided on one end of the said body. Grooves which extend along the axial direction are provided on the outside of the said prongs. A clamping ring which is inserted in the said grooves is mounted on the free end of the said prongs.

## Description

### Field of the Invention

The present invention relates to an intramedullary pin, more particularly to an intramedullary pin that can pass directly into a fractured bone and connect securely with the fracture.

### Description of the Related Art

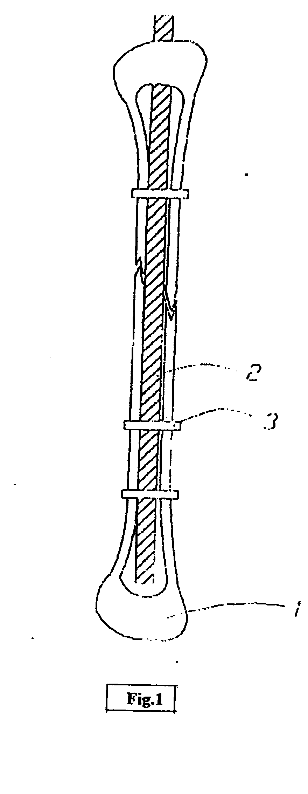

Please refer to FIG 1 for a traditional method for connecting a fractured bone 1. The method is nothing more than passing an intramedullary pin 2 into an intramedullary cavity. Since the intramedullary pin 2 is usually straight, therefore when the intramedullary pin 2 enters into the intramedullary cavity, an accessory pin 3 must be installed onto the surface of the intramedullary cavity along its axial direction first to prevent the intramedullary pin 2 from falling out of the intramedullary cavity. In the meantime, examinations by X-ray instruments are required before performing the surgical operation as to precisely press the accessory pin 3 against the intramedullary pin 2 and a fixation element is required to connect the fracture in order to accurately connect the fractured bones. Such arrangement not only increases the time of the surgical operation, but also makes the newly grown blood vessels and callous formations unable to cover the bone effectively or connect the fractured bone. Such method has a significant deficiency in its application and increases the pain of the patient. The foregoing drawbacks have been bothering medical staffs and the patients for long.

To overcome the foregoing drawbacks, an intramedullary pin having a split-end is invented. Although the intramedullary pin can press its prong into the intramedullary cavity, so that the intramedullary pin can be fixed into the intramedullary cavity directly when the intramedullary pin enters into the intramedullary cavity, yet the angle of the prong at the front end of the intramedullary pin is too small, which is unable to achieve the effect of pressing the intramedullary cavity. If the angle of the prong is too large, then it is not easy for the intramedullary pin to enter into another end of the intramedullary cavity when it travels. Therefore, it requires using a surgical forceps to clamp the intramedullary pin, but the surface of the intramedullary pin is very smooth. It is difficult to clamp the intramedullary pin by the forceps, and thus makes the operation difficult and inconvenient.

### Summary of the Invention

Therefore it is the primary objective of the present invention to provide a technical solution for the foregoing problems as well as an intramedullary pin that can pass directly into a fractured bone and connect securely with the fracture.

The technical measures taken by the present invention is to provide an intramedullary pin that comprises a body, characterized in that a prong is disposed at one end of the body and extended in different directions with an open end; a groove is disposed on an external side of the prong and extended along its axial direction; and a clamping ring is embedded in the groove. With the implementation of the foregoing solution, it is obvious that the present invention achieves the following beneficial effects. The structure of the present invention is feasible structure and the idea is great. When the intramedullary pin is applied, the body enters into a fractured intramedullary cavity from one end and moves forward to the other end of the intramedullary cavity. When the prong on the body is exposed from the fractured position, the ball-head bone holding forceps is embedded into the groove, such that when the prong moves forward, the ball-head bone holding forceps will move along the groove to push the prong to another end of the intramedullary cavity and the prong remains closed since it is clamped by the ball-head bone holding forceps. Therefore, the prong can successfully enter into another end of the intramedullary cavity. The prong will be opened slowly after it reaches another end of the intramedullary cavity, so that the prong will be fixed without falling out, and thus further achieves the purpose of connecting the fractured intramedullary cavity. Further, a clamping ring embedded in the groove is disposed at a free end of the prong, and thus when the prong enters into the fractured intramedullary cavity from one end, the prongs will be aligned next to each other by the restriction of the clamping ring, and the body can successfully move forward from that end of the intramedullary cavity to the other end of the intramedullary cavity. Further, the groove has a depth tapered from one end of the open prong to the other end, such that when the ball-head bone holding forceps is embedded in the groove, the ball-head holding forceps can be detached naturally from the groove by the change of the depth of the tapered groove. The present invention is worthy for its promotion.

To make it easier for our examiner to understand the objective of the invention, its structure, innovative features, and performance, we use a preferred embodiment together with the attached drawings for the detailed description of the invention.

### BRIEF DESCRIPTION OF THE DRAWINGS

FIG 1 is an illustrative of a prior-art intramedullary pin.

FIG 2 is an illustrative of the intramedullary pin according to the present invention.

FIG 3 is an illustrative view of the intramedullary pin entering the intramedullary cavity according to the present invention.

FIG 4 is an illustrative view of the intramedullary pin being protruded and exposed from the intramedullary cavity according to the present invention.

FIG 5 is an illustrative view of the ball-head bone holding forceps clamping the intramedullary pin according to the present invention.

FIG 6 is an illustrative view of the ball-head bone holding forceps clamping the intramedullary pin when it is implemented according to the present invention.

FIG 7 is an illustrative view of the intramedullary pin entering into another intramedullary cavity according to the present invention.

FIG 8 is an illustrative view of the intramedullary pin connecting the fractured intramedullary cavity according to the present invention.

### DETAILED DESCRIPTION OF THE PREFERRED EMBODIMENTS

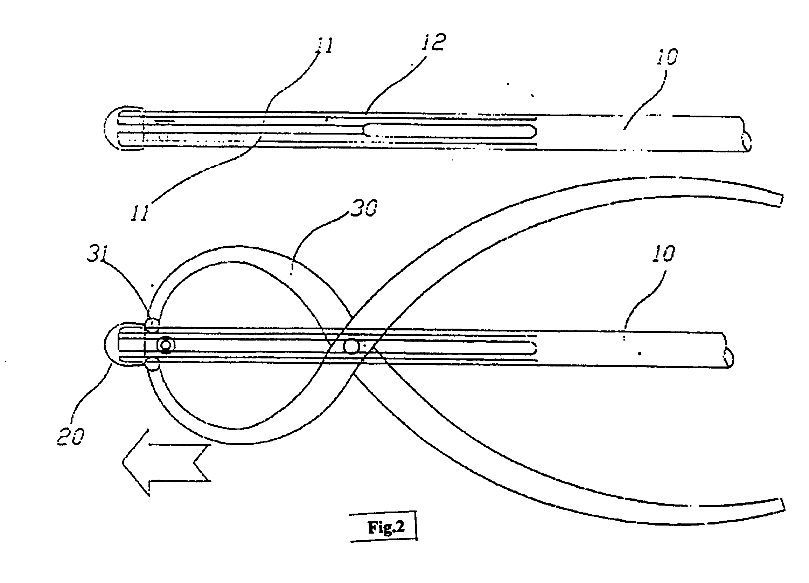

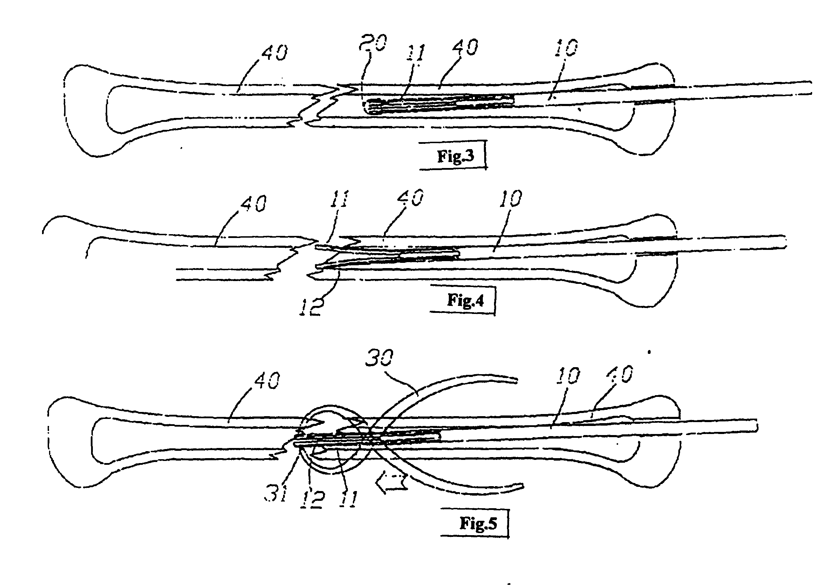

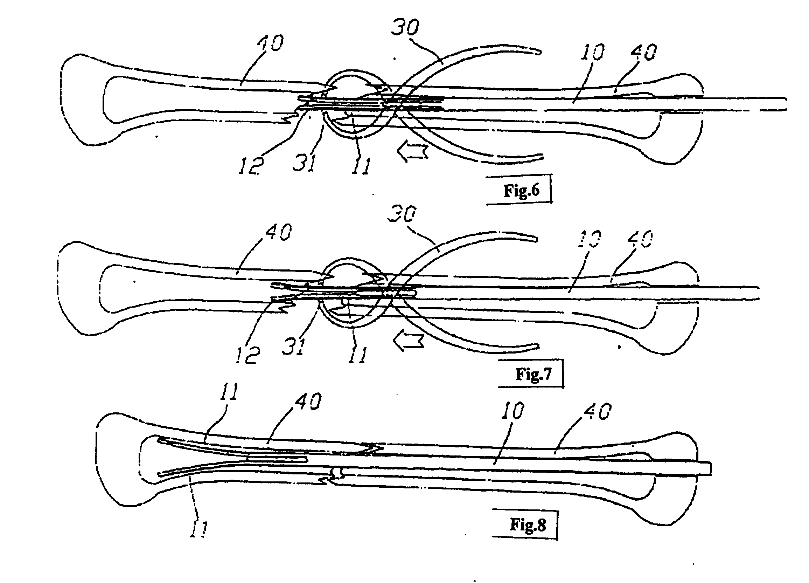

Please refer to FIG 2 for an intramedullary pin according to the present invention. The intramedullary pin is a device for fixing and connecting fractured intramedullary cavity, and such device comprises a body 10 which is a rod used in this embodiment; a prong 11 being disposed at one end of the body 10 and extended in different directions with an open end; a groove 12 being disposed on an external side of the prong 11 and extended along its axial direction, and the groove 12 is tapered from the open end of the prong 11 to another end; and a clamping ring 20 being coupled to a free end of the prong 11, and the clamping ring 20 is a C-shaped ring adopted in this embodiment, and the clamping ring 20 is embedded into the groove 12, such that the prongs 12 align with each other under the restriction of the clamping ring 20. When the intramedullary pin is applied, the body 20 enters into an intramedullary cavity 20 from one end and moves forward to the other end of the intramedullary cavity 20 as shown in FIG 3 until the prong 12 on the main body 10 is exposed at the fracture position. When the prong 12 is exposed at that position, the clamping ring 20 is removed from the prong 11 as shown in FIG 4. In the meantime, the ball-head bone holding forceps 30 having a ball-shaped clamping head 31 is embedded into the groove 12 as shown in FIG 5, such that the ball-shaped clamping head 31 of the ball-head bone holding forceps 30 will move along the groove 12 and will not fall out. When the prong 11 is pushed to another end of the intramedullary cavity 40, the prong 11 remains closed since it is clamped by the clamping head 31 of the ball-head bone holding forceps 30 as shown in FIG 6. Therefore, when the prong 11 successfully enters into another end of the intramedullary cavity 40 and the ball-shaped head 31 of the ball-head bone holding forceps 30 is embedded in the groove 12, the prong 11 will detach naturally from the groove 12 by means of the tapered groove 12. The prong 11 will be opened slowly to a predetermined angle after it enters another end of the intramedullary cavity 40, so that the prong 12 will be fixed by the support of the opened prong 12 on the body 10 and will not fall out from that end of the intramedullary cavity 40. Thus the present invention can connect a fractured intramedullary cavity 40 as shown in FIGS. 7 and 8.

In summation of the description above, the present invention overcomes the shortcomings of the prior-art and enhances the performance than the conventional structure and further complies with the patent application requirements and is submitted to the Patent and Trademark Office for review and granting of the commensurate patent rights.

While the invention has been described by way of example and in terms of a preferred embodiment, it is to be understood that the invention is not limited thereto. To the contrary, it is intended to cover various modifications and similar arrangements and procedures, and the scope of the appended claims therefore should be accorded the broadest interpretation so as to encompass all such modifications and similar arrangements.

## Claims

1. An intramedullary pin comprising a body, **characterized in that** a prong being disposed at one end of said body and extended in different directions with an open end; a groove being disposed on an external side of said prong and extended along its axial direction; and a clamping ring being embedded in said groove.

2. The intramedullary pin of claim 1, wherein said prong of said body is clamped and aligned side by side with a ball-head bone holding forceps.

3. The intramedullary pin of Claims 1 or 2, wherein said groove has a depth tapered from one end of said open prong to the other end.
